(19) Europäisches Patentamt
European Patent Office
Office européen des brevets

(11) EP 2 787 050 A1

(12) **EUROPEAN PATENT APPLICATION**
published in accordance with Art. 153(4) EPC

(43) Date of publication:
**08.10.2014 Bulletin 2014/41**

(21) Application number: **12852762.9**

(22) Date of filing: **30.11.2012**

(51) Int Cl.:
*C09K 3/00* (2006.01)
*A23L 1/03* (2006.01)
*A61K 8/02* (2006.01)
*A61K 8/55* (2006.01)
*A61K 8/67* (2006.01)
*A61K 9/06* (2006.01)
*A61K 47/14* (2006.01)
*A61K 47/22* (2006.01)
*A61K 47/24* (2006.01)
*C09D 5/04* (2006.01)
*C09D 7/12* (2006.01)
*C09D 201/00* (2006.01)
*C10M 141/10* (2006.01)
*C10M 129/20* (2006.01)
*C10M 137/04* (2006.01)

(86) International application number:
**PCT/JP2012/081125**

(87) International publication number:
**WO 2013/081120 (06.06.2013 Gazette 2013/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2011 JP 2011262226**

(71) Applicant: **Nihon University**
**Tokyo 102-8275 (JP)**

(72) Inventors:
- **HASHIZAKI, Kaname**
  **Tokyo 102-8275 (JP)**
- **SAITO, Yoshihiro**
  **Tokyo 102-8275 (JP)**
- **TAGUCHI, Hiroyuki**
  **Tokyo 102-8275 (JP)**

(74) Representative: **Høiberg A/S**
**St. Kongensgade 59 A**
**1264 Copenhagen K (DK)**

(54) **LECITHIN-ORGANOGEL-FORMING AGENT**

(57) An organogelling agent for forming reverse worm-like micelles having high safety to living bodies and the environment, favorable feel of use, and a high gelating ability and a thickened gel composition comprising the organogelling agent are provided. Lecithin and ascorbic acid or an ascorbic acid derivative act as an organogelling agent to form reverse worm-like micelles of a variety of oils. A thickened gel composition having a reverse worm-like micelle structure can be prepared with a ternary system of lecithin, ascorbic acid or an ascorbic acid derivative, and a variety of oils.

FIG.1

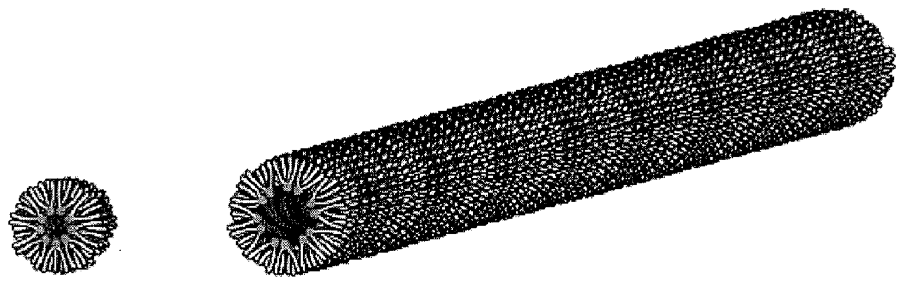

The reverse spherical micelle The reverse worm-like micelle

EP 2 787 050 A1

## Description

### TECHNICAL FIELD

The present invention relates to a lecithin organogelling agent that solidifies by thickening or gelating oils, such as animal and vegetable oils, mineral oils, hydrocarbons, and fatty acid esters.

### BACKGROUND ART

Organogel formers for thickening or gelating oils, such as animal and vegetable oils, mineral oils, hydrocarbons, and fatty acid esters, have been extensively used in various fields in cosmetics, medicines, foods, coating materials, inks, and lubricants. Properties essential for these organogel formers are to facilitate the gelation of a target oil by addition of a small amount of the organogelling agent and to stabilize the resulting gel for a long term. Some applications require high safety to human bodies, generation of thixotropic gel, and a favorable touch of the produced gel.

Known organogelling agents include low molecular gelling agents, such as 1,2,3,4-dibenzylidene-D-sorbitol, 12-hydroxystearic acid, and amino acid derivatives; and high molecular gelling agents, such as polyacrylic acid derivatives and dextrin derivatives. The molecules in a low molecular gelling agent self-assemble in oil to form a large network structure that inhibits the flow of the oil to gelate the oil. The molecules in a high molecular gelling agent are entangled into a complicated network structure to gelate the oil.

A low molecular gelling agent 1,2,3,4-dibenzylidene-D-sorbitol decomposes to generate benzaldehyde although this compound can gelate a variety of oils. Such a compound is not suitable for practical use in terms of safety. 12-Hydroxystearic acid commercially available as a gelling agent for a waste cooking oil has little or no thixotropy. Gelling agents made of amino acid derivatives have low solubility in oils, and need a complicated operation to dissolve the gelling agents in the oils, such as heating at high temperatures and agitation for a long time. Such an operation may denature other components compounded in the gel. For a high molecular gelling agent made of a dextrin derivative, addition in a large amount is inevitable for gelation. The high molecular gelling agent also causes "stickiness" inherent in the polymer, leading to bad feel of use. For a gelling agent made of a polyacrylic acid derivative, addition of a small amount results in favorable thickening or gelation. The gel applied to the skin, however, has the "stickiness" inherent in the polymer and gives bad feel of use.

To solve these problems, for example, Patent Document 1 discloses a gel emulsion including one or two natural surfactants, such as lecithin and sucrose fatty acid esters, a higher alcohol, glycerol, and an oil. Unfortunately, the gel emulsion is easy to drip and difficult to handle due to its low elasticity. In addition, the effect of gelation is not attained if one of the higher alcohol and glycerol is eliminated. No organogelling agent has not yet been developed that has all the advantageous characteristics, such as high safety to living bodies and the environment, high gelating ability, favorable feel of use, and ease of handling.

Some cases have been reported on the gelation of oils with reverse worm-like micelles (Non Patent Documents 1 to 3). The reverse worm-like micelle is one of the self-assemblies formed of surfactants, and it is known that the reverse worm-like micelles form a network structure in oil to cause gelation. The reverse worm-like micelle having an internal hydrophilic environment can encapsulate water-soluble drugs or enzymes. The reverse worm-like micelle has such unique characteristics, which are not found in the organogelling agent described above.

Non Patent Document 1 discloses ternary systems of lecithin, water, and a variety of oils, which are typical systems for forming the reverse worm-like micelle. For substitutions for water, ethylene glycol (Non Patent Document 2), formamide (Non Patent Document 2), and bile acid salts (Non Patent Document 3) have been reported. Typically, lecithin forms reverse spherical or ellipsoidal micelles in oil. When a small amount of water or any other solvent is added to this system, hydrogen binds to the phosphate group of lecithin to decrease the interfacial curvature of the molecular assembly, so that the reverse worm-like micelles grow.

Unfortunately, drugs susceptible to hydrolysis cannot be encapsulated in these typical reverse worm-like micelles because these micelles are composed of lecithin, water, and a variety of oils. Ethylene glycol and formamide as substitutions for water cannot be used for human bodies because these are strongly irritant to skin, eyes, and mucous membranes. Although the bile acid salts are surfactants derived from living bodies, the bile acid salts adhering to skin or eyes may cause inflammation.

The present inventors have proposed organogelling agents for forming reverse worm-like micelles composed of lecithin and sucrose fatty acid ester (Patent Document 2), lecithin and saccharide (Patent Document 3), lecithin and urea (Patent Document 4), as organogelling agents having all the advantageous characteristics, such as high safety to living bodies and the environment, high gelating ability, favorable feel of use, and ease of handling. Unfortunately, agents containing sucrose fatty acid esters or saccharides have odors inherent in sugars, and agents containing urea are not suitable for food products.

RELATED ARTPATENT DOCUMENT

Patent Document 1: Japanese Patent Application Laid-Open No. 5-4911
Patent Document 2: WO 2010/082487
Patent Document 3: WO 2010/122694
Patent Document 4: Japanese Patent Application Laid-Open No. 2010-270299

NON-PATENT DOCUMENT

Non Patent Document 1: P. L. Luisiet al., Colloid & Polymer Science, vol. 268, p. 356 (1990)
Non Patent Document 2: Yu. A. Shchipunov, Colloids and Surfaces A, vol. 183-185, p. 541 (2001)
Non Patent Document 3 : S. H. Tung et al., Journal of the American Chemical Society, vol. 128, p. 5751 (2006)

SUMMARY OF INVENTION

An object of the present invention is to provide an organogeling agent for forming reverse worm-like micelles, the organogelling agent having all the advantageous characteristics required for a thickened gel composition, such as high safety to living bodies and the environment, favorable feel of use, and high gelating ability, and a thickened gel composition including the organogelling agent.

SOLUTION TO PROBLEM

The present inventors, who have conducted extensive research to solve the problems, have found that a mixture of lecithin and ascorbic acid or an ascorbic acid derivative acts as an organogelling agent for forming reverse worm-like micelles in a variety of oils, that thickened gel compositions having a reverse worm-like micelle structure can be prepared with ternary systems of lecithin, ascorbic acid or an ascorbic acid derivative, and a variety of oils, and that the thickened gel compositions have high safety to human bodies and the environment, high gelation performance, and long-term stability, and have attained the solution.
The present invention provides:

Aspect [1] An organogelling agent for forming reverse worm-like micelles, including lecithin (a) and ascorbic acid or an ascorbic acid derivative (b).
Aspect [2] The organogelling agent according to Aspect [1], wherein the mixture contains ascorbic acid or ascorbic acid derivative (b) in an amount of 0.1 to 35 mass% to the total mass of lecithin (a) and ascorbic acid or ascorbic acid derivative (b).
Aspect [3] A thickened gel composition, including the organogelling agent according to Aspect [1] or [2] and an oil component (c), wherein the thickened gel composition contains reverse worm-like micelles.
Aspect [4] The thickened gel composition according to Aspect [3], wherein the thickened gel composition is at least one composition selected from a cosmetic composition, a medicine composition, a food composition, a coating composition, an ink composition, and a lubricant composition.
Aspect [5] The thickened gel composition containing oil component (c) according to Aspect [3] or [4], wherein the thickened gel composition contains 1 to 70 mass% organogelling agent.
Aspect [6] A process for preparing an organogelling agent for forming reverse worm-like micelles, including: dissolving lecithin (a) and ascorbic acid or an ascorbic acid derivative (b) in an organic solvent, and evaporating the organic solvent. Aspect [7] A process for preparing a thickened gel composition containing reverse worm-like micelles, including: dissolving lecithin (a) and ascorbic acid or an ascorbic acid derivative (b) in an organic solvent, and evaporating the organic solvent to prepare an organogelling agent; and mixing an oil component (c) with the organogelling agent.

EFFECTS OF INVENTION

The organogelling agent for forming reverse worm-like micelles according to the present invention is of an environment-friendly type having high safety to living bodies and the environment, and can also gelate the target oil by addition of a small amount. The organogelling agent can be widely used in a variety of cosmetics, medicines, foods,

coating materials, inks, and lubricants. The thickened gel composition prepared from the organogelling agent has thixotropy and has advantageous effects, such as a reduction in dripping, ease of handling, and long-term stability over one year. The transparency of the thickened gel composition can be controlled depending on its application, for example, to be transparent, translucent, or opaque by setting preparative conditions on the organogelling agent and the thickened gel composition. Furthermore, unlike traditional thickened gel compositions, the thickened gel composition according to the present invention has a hydrophilic environment inside the reverse worm-like micelle structure and can encapsulate water-soluble components, drugs, and enzymes.

BRIEF DESCRIPTION OF DRAWINGS

Fig. 1 illustrates a reverse micelle structure.
Fig. 2 is a diagram illustrating a small-angle X-ray scattering curve.

DESCRIPTION OF EMBODIMENT

The present invention will now be described. The organogelling agent according to the present invention can thicken or gelate a variety of oil components to form reverse worm-like micelles. The organogelling agent according to the present invention contains lecithin (a) and ascorbic acid or an ascorbic acid derivative (b). When the organogelling agent is mixed with an oil component (c), oil component (c) is thickened or gelated into a thickened gel composition having a reverse worm-like micelle structure.

The reverse worm-like micelle is a self-assembly of surfactant molecules. The surfactant is an amphiphilic substance having a hydrophilic group and a hydrophobic group in its molecule, and surfactant molecules form a self-assembly in water or oil depending on the balance of these groups. As illustrated in Fig. 1, the reverse worm-like micelle cylindrically grown from a reverse spherical micelle forms a temporary network structure to form a highly viscoelastic gel. The reverse worm-like micelle has an internal hydrophilic environment, and can encapsulate water-soluble components, drugs, and enzymes.

The present inventors, who have conducted extensive research on the reverse worm-like micelle, have found that the substance inducing the growth of the reverse worm-like micelle needs to satisfy two conditions: the substance has two or more functional groups that hydrogen-bond to the phosphate groups of lecithin, and the substance is slightly hydrophobic. The present inventors have paid attention to ascorbic acid or an ascorbic acid derivative as such an inducing substance, and have found that the reverse worm-like micelle can be formed from lecithin, ascorbic acid or an ascorbic acid derivative, and an oil.

The present inventors observed the structure of the prepared thickened or gelated product, specifically the thickened or gelated product prepared by mixing lecithin with ascorbic acid and n-decane. The thickened or gelated product is transparent, and has no crystal structure. The thickened or gelated product is optically isotropic, and exhibits no characteristic pattern in a polarized image. The small-angle X-ray scattering (SAXS) curve does not have any distinct diffraction peak. This suggests that the thickened or gelated product has no regular structure, and the thickened gel composition according to the present invention contains reverse worm-like micelles.

The transparency of the thickened gel composition according to the present invention varies according to the oil to be thickened or gelated. For example, a transparent oil produces a transparent thickened gel composition due to the formation of the reverse worm-like micelles.

The SAXS measurement was performed with a Nano-STAR available from Bruker AXS K.K. with a CuK$\alpha$ X-ray source at an output of 45 kV/120 mA. The SAXS measurement was performed at 25°C.

Fig. 2 shows a scattering curve of the thickened gel composition (lecithin: 2 mass%, ascorbic acid: 0.26 mass%, n-decane: 97.74 mass%) obtained by small-angle X-ray scattering (relationship between the scattering intensity I(q) and the scattering vector q). In the drawing, $q = (4\pi/\lambda)\sin\theta$, where $\theta$ indicates scattering angle and $\lambda$ indicates wavelength of the X-ray. In Fig. 2, the scattering curve shows no distinct diffraction peak. This suggests that the thickened gel composition has no regular structure. In the scattering curve, the slope of the log-log plot in the low-q region is -1. This suggests the existence of long rod-like particles, that is, reverse worm-like micelles. From the cross-section plot based on Expressions (1) and (2), the cross sectional radius (r) of the reverse worm-like micelle is determined as 1.8 nm.

$$\ln qI(q) = \ln qI(0) - (1/2)R_c^2 q^2 \qquad (1)$$

$$r = \sqrt{2} R_c \qquad (2)$$

where Rc represents a radius of gyration of the cross section. Although the exact length of the reverse worm-like micelle cannot be calculated due to the limited range in the measurement, the simulation using Expression (3), a theoretical scattering function, under presumption of rod-like particles (Calculated curve in the diagram) shows that the length (t) of the reverse worm-like micelle is longer than 50 nm.

$$I(q) = \int_0^{\pi/2} \frac{\sin^2(q(t/2)\cos\theta)}{q^2(t/2)^2\cos^2\theta} \frac{4J_1^2(qr\sin\theta)}{q^2r^2\sin^2\theta} \sin\theta d\theta \qquad (3)$$

where $J_1$ represents a primary Bessel function. The state of gelation and physical properties of the thickened gel composition prepared by thickening or gelating the oil component with the organogelling agent according to the present invention suggests the formation of reverse worm-like micelles.

Lecithin (a) in the organogelling agent according to the present invention is a lipid product containing phosphatidylcholine as the main component. It is known that lecithin is widely found in living bodies such as animals, plants, and microorganisms, and is contained much in livers, egg yolks, soybean, and yeasts. Typical examples of lecithin include egg yolk lecithin and soy lecithin, and use of these is preferred. These lecithins may be used alone or in combination. Lecithin preferably contains approximately 55 to 99% by weight phosphatidylcholine. Natural lecithin is present in only an L-$\alpha$ form, and lecithins in other forms can also be used. Natural lecithin, which is unstable and readily oxidized, may be hydrogenated by a known method for use. In the present invention, the term "lecithin" also includes such hydrogenated lecithins.

Phosphatidylcholine refers to an ester produced by a reaction of glycerol with at least one unsaturated fatty acid and phosphoric acid. During the reaction, a proton of the phosphoric acid is replaced with choline as an amino functional group. In the present invention, the term "phosphatidylcholine" also includes phosphatidylcholines having hydrogenated unsaturated bond.

In the present invention, phosphatidylcholine is defined by Formula (I), where $R_1$ and $R_2$ each independently represent an aliphatic hydrocarbon group derived from (corresponding to) a saturated or unsaturated fatty acid having 4 to 24 carbon atoms (that is, saturated or unsaturated aliphatic group having 3 to 23 carbon atoms) ; $R_1$ and $R_2$ may be linear or branched, or may be substituted by one or more hydroxyl functional groups and/or amine functional groups; X represents a choline residue. The phosphatidylcholine may be one selected from compounds represented by Formula (I), or may be a mixture of the compounds.

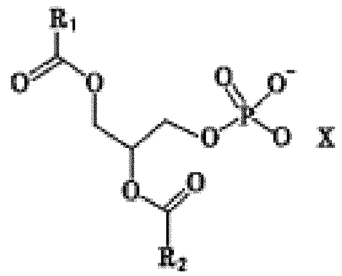

(I)

In one embodiment according to the present invention, fatty acids ($R_1COOH$, $R_2COOH$) for $R_1$ and $R_2$ are selected from butyric acid, caproic acid, caprylic acid, capric acid, caproleic acid, lauric acid, lauroleic acid, myristic acid, myristoleic acid, palmitic acid, palmitoleic acid, stearic acid, oleic acid, linoleic acid, linolenic acid, arachidic acid, isostearic acid, dihydroxystearic acid, and ricinoleic acid.

"Natural" or "synthetic" non-hydrogenated phosphatidylcholine (PC) can be suitably used for the composition according to the present invention.

"Natural" PC can be extracted from animal sources (such as eggs) or plant sources (such as soybean and sunflower). Non-hydrogenated phosphatidylcholine extracted from a natural product, such as soybean, typically contains fatty acids, which can esterify glycerol, i.e., palmitic acid, stearic acid, palmitoleic acid, oleic acid, linoleic acid, linolenic acid, and probably $C_{20}$ to $C_{22}$ fatty acids.

The organogelling agent according to the present invention contains component (b), i.e., ascorbic acid or an

ascorbic acid derivative. L-ascorbic acid (vitamin C, hereinafter referred to as "ascorbic acid"), one of the water-soluble vitamins, generates collagen in human bodies and acts as an important antioxidant. L-ascorbic acid has widely been used in medical supplies, foods, dietary supplements, and cosmetics because of its little or no toxicity to human bodies, high safety, and low industrial cost and availability. Any ascorbic acid or ascorbic acid derivative soluble in organic solvents used to prepare thickened gel compositions can be used.

The ascorbic acid derivative includes isomers of ascorbic acid, such as ascorbic acid alkylethers, ascorbic acid alkylesters, ascorbic acid glucosides, and erythorbic acid, and derivatives of the isomers. Among these, preferred ascorbic acid alkylether is 3-O-alkyl ascorbic acid. 3-O-alkyl ascorbic acid has an alkyl group having 1 to 22 carbon atoms. The alkyl group having 1 to 22 carbon atoms may be linear, branched, or cyclic, and examples thereof include methyl, ethyl, n-propyl, isopropyl, n-butyl, isobutyl, sec-butyl, tert-butyl, pentyl, hexyl, octyl, decyl, dodecyl, tetradecyl, hexadecyl, octadecyl, behenyl, cyclopropyl, cyclobutyl, cyclopentyl, cyclohexyl, and cyclooctyl.

The organogelling agent according to the present invention may contain lecithin (a) and the ascorbic acid or an ascorbic acid derivative (b) in any mixing ratio in which the reverse worm-like micelles can be formed. The content of lecithin (a) is 1 to 70 mass%, preferably 1.5 to 45 mass%, more preferably 2 to 25 mass%, and the content of the ascorbic acid or an ascorbic acid derivative (b) is 0.1 to 20 mass%, preferably 0.15 to 15 mass%, more preferably 0.2 to 10 mass%. The ratio of the mass of the ascorbic acid or an ascorbic acid derivative (b) to the total mass of the organogelling agent varies depending on oil component (c). In the mass ratio (mass%) calculated from component (b)/(component (a) + component (b)), the experimental lower limit is 0.1 mass%, preferably 1 mass%, more preferably 7 mass%, and the experimental upper limit is 35 mass%, preferably 30 mass%, more preferably 25 mass%.

Lecithin (a) and ascorbic acid or an ascorbic acid derivative (b) are dissolved in any suitable organic solvent, and then the organic solvent is evaporated to prepare the organogelling agent according to the present invention. Oil component (c) is mixed to the organogelling agent to prepare a thickened gel composition containing reverse worm-like micelles.

In the present invention, any oil component (c) can be gelated, and the oil component (c) includes oils, such as animal and vegetable oils, mineral oils, hydrocarbons, and fatty acid esters. The oil component (c) may be composed of a polar oil alone, a nonpolar oil alone, or a mixture of a polar oil and a nonpolar oil. Specific examples thereof include fish oils and animal oils, such as fish oil, liver oil, whale oil, tallow, lard, horse oil, and lanolin; vegetable oils, such as coconut oil, palm oil, cocoa butter, olive oil, rapeseed oil, and linseed oil; hydrocarbons, such as liquid paraffin, isoparaffin, kerosene, heavy oil, n-decane, isooctane, n-octane, n-heptane, n-hexane, and cyclohexane; higher fatty acids, such as lauric acid, palmitic acid, stearic acid, oleic acid, and behenic acid; and fatty acid esters, such as isopropyl myristate, 2-octyldodecyl myristate, and isopropyl palmitate. These oils may be used alone or in combination.

Any additive component may be dissolved, dispersed, emulsified, suspended, or mixed in the oil to be organogelated within a concentration not inhibiting thickening or gelation of the oil. Examples of such an additive component include surfactants, ultraviolet absorbers, moisturizing agents, antiseptic agents, preservative agents, bactericides, antioxidants, fluidity improvers, fragrances, dyes, enzymes, and bioactive substances according to the applications, such as cosmetics, medicines, foods, coating materials, inks, and lubricants, and specifically include organic compounds or inorganic compounds, such as titanium oxide, talc, mica, and water.

In gelation of oil component (c) with the organogelling agent according to the present invention comprising lecithin (a) and ascorbic acid or an ascorbic acid derivative (b), the organogelling agent can be mixed with the oil component in any amount capable of forming reverse worm-like micelles through thickening and gelation of oil component (c). If the content of lecithin (a) is 1 to 70 mass%, preferably 1.5 to 45 mass%, more preferably 2 to 25 mass% and if the content of ascorbic acid or an ascorbic acid derivative (b) is 0.1 to 20 mass%, preferably 0.15 to 15 mass%, more preferably 0.2 to 10 mass%, the content of oil component (c) can be 30 to 99 mass%, preferably 50 to 98 mass%, more preferably 70 to 97 mass%.

In the proportion of the organogelling agent to the thickened gel composition calculated from (component (a) + component (b))/(component (a) + component (b) + component (c)) (mass%), the lower limit is 1 mass%, preferably 1.5 mass%, more preferably 2 mass%, and the upper limit is 70 mass%, preferably 50 mass%, more preferably 30 mass%. The organogelling agent within this range is mixed with the oil component, and the mixture is left to prepare a thickened gel composition. The strength of the gel can be adjusted according to the content of the organogelling agent and the mixing ratio of lecithin (a) to ascorbic acid or an ascorbic acid derivative (b).

The thickened gel composition according to the present invention comprises the organogelling agent contains lecithin (a) and ascorbic acid or an ascorbic acid derivative (b), and can be gelated to have a reverse worm-like micelle structure by adding a variety of oil components (c) and optional additive components to the organogelling agent, and uniformly dissolving these components. The thickened gel composition can be prepared, for example, as follows: Necessary amounts of lecithin (a) and ascorbic acid or an ascorbic acid derivative (b) are sealed in a container, and an organic solvent is added; component (a) and component (b) are completely dissolved by agitation, and the organic solvent is completely evaporated under reduced pressure; a necessary amount of oil component (c) is added to the product, and is agitated overnight; and then the container is optionally settled in a thermostat for several days to stabilize

the product. Alternatively, the thickened gel composition can be prepared by dissolving a mixture of lecithin (a), ascorbic acid or an ascorbic acid derivative (b), and a variety of oil components (c) by heating, and cooling the mixture to room temperature. The heating may be performed at any temperature that facilitates dissolution of the mixture, preferably at 50 to 80°C. To prevent oxidation of the lecithin and the ascorbic acid or an ascorbic acid derivative, the heating is performed preferably under an atmosphere of an inert gas, such as nitrogen. The thickened gel composition is produced immediately after addition, mixing, and agitation of the components. The long-term agitation and settlement of the gel product for stabilization are optional, and are omittable depending on cases.

A preferred process for preparing the thickened gel composition according to the present invention comprises dissolving lecithin (a) and ascorbic acid or an ascorbic acid derivative (b) in an organic solvent, and evaporating the organic solvent to prepare an organogelling agent; and mixing oil component (c) with the resultant organogelling agent to prepare a thickened gel composition containing reverse worm-like micelles.

Examples of the organic solvent used in preparation of the organogelling agent and the thickened gel composition include lower alcohols, such as methanol, ethanol, propanol, and butyl alcohol; polyhydric alcohols, such as ethylene glycol and propylene glycol; ketones, such as acetone, 2-butanone, and cyclohexanone; ethers, such as tetrahydrofuran and 1,4-dioxane; esters, such as ethyl acetate and butyl acetate; amides, such as N,N-dimethylformamide and N,N-dimethylacetamide; halogenated hydrocarbons, such as chloroform, carbon tetrachloride, dichloromethane, dichloroethane, isopropyl bromide, ethyl bromide, dichlorobenzene, tetrachloroethane, trichloroethane, trichloroethylene, and tetrachloroethylene; and water. Among these, use of lower alcohols is preferred. These organic solvents may be used alone or in combination.

The thickened gel composition according to the present invention can be used as a variety of gel products at normal temperature, such as cleansers, cosmetics, medicines, foods, deodorants, bath essences, and fragrances. Among these, especially applications in cleansers, cosmetics, medicines, and foods are suitable. Examples of the cleansers include food cleansers, dish cleansers, kitchen cleansers, facial cleansers, body soaps, shampoos, and hair rinses. Examples of the cosmetics include creams, milky liquids, lotions, cleansing lotions, bath cosmetics, moisture cosmetics, blood circulation promoting and massage lotions, face and body packs, and hair and scalp cosmetics. Examples of the medicines include ointments, molded poultices, sustained-release formulation bases, transdermal absorption formulations, carriers in drug delivering systems, and gels for electrophoresis.

The cosmetics may contain typical components used in typical cosmetics. Examples thereof include fragrances, dyes, preservative agents, antioxidants, antiinflammatory agents, ultraviolet absorbers, ultraviolet light reflecting agents, and pH adjusters. Various optional active ingredients, such as hyaluronic acid, allantoin, vitamins, amino acids, and placenta extracts, may be properly contained. These may be used alone or in combination.

The thickened gel composition including the organogelling agent according to the present invention and a variety of oils has a reverse worm-like micelle structure, which has an internal hydrophilic environment and can encapsulate water-soluble components, drugs, and enzymes. The reverse worm-like micelles are extremely fine molecular assemblies in nanometers. In such a reverse worm-like micelle structure, polar groups in the lecithin molecules and the ascorbic acid or ascorbic acid derivative molecules face inwardly, and hydrophobic groups of these molecule face outwardly to form numerous worm-like assemblies. These worm-like assemblies have internal hydrophilic environments, that is, small water pools (aqueous phases). Examples of the water-soluble substances that can be encapsulated include drug components, such as skin whitening agents, antiinflammatory agents, antibacterial agents, hormones, vitamins, a variety of amino acids and their derivatives, enzymes, antioxidants, and hair growers.

The thickened gel composition including the organogelling agent according to the present invention and a variety of oils is put into contact with a water-soluble substance, drug, or enzyme that is less soluble or insoluble in the oils directly or in a form of an aqueous solution, and the components are mixed by stirring. The water-soluble substance can be trapped in the reverse worm-like micelles to be dissolved in the thickened gel. The transparency of the prepared thickened gel composition can be controlled according to the organogelling agent to be used and the preparative conditions on the thickened gel composition to be transparent, translucent, or opaque depending on its application.

The organogelling agent according to the present invention has a high gelating ability to various oils to generate a stable gel for a long term by addition of a small amount of the organogelling agent. In particular, gels composed of higher hydrocarbons have high thixotropy, and have no deficits in a touch, such as "stickiness." The organogelling agent according to the present invention is also of an environment-friendly type composed of lecithin and ascorbic acid or an ascorbic acid derivative having high safety to human bodies and the environment and high biodegradability.

The organogelling agent according to the present invention can also be used in the fields of quasi-drugs other than those described above, inks, coating materials, and lubricants, in the processing fields of plastics, rubbers, and metals, and in the fields of agriculture, fishery, and treatment of waste oils.

## EXAMPLES

The present invention will now be described in more detail according to non-limiting Examples. The amount of

each component is expressed by mass% unless otherwise specified.

[Characterization of thickened gel composition]

The viscosity was measured under a constant temperature of 25°C with a rotary rheometer (RheoStress 600, available from HAAKE) provided with cone plates (diameter: 60 mm, 35 mm, cone angle: 1°, 4°) and a Peltier temperature controller. To prevent evaporation of the solvent, a solvent trap was used during the measurement. Specifically, a sample was disposed between the cone plate and a sample stand. While the cone plate was being rotated in a predetermined direction, the shear rate was gradually increased. A shear stress was determined at each shear rate, and a zero shear viscosity was calculated from the relation: viscosity = shear stress/shear rate.

The state of thickening or gelation was evaluated as follows:

AA: gelation (zero shear viscosity: 100 Pa·s or more);
A: thickening (zero shear viscosity: 10 Pa·s or more and less than 100 Pa·s);
F: insufficient thickening or gelation (zero shear viscosity: less than 10 Pa·s)

[Transparency of thickened gel composition]

The transparency of the gel was evaluated as follows. The thickened gel composition was sealed in a sample vial having a diameter of 27.5 mm and a height of 70 mm, and was preserved for one month in a thermostat set at 25°C. The thickened gel composition was then visually evaluated as AA: transparent, A: translucent, C: opaque, and F: separated into two phases.

[Examples 1 to 11]

(Compounding of thickened gel composition)

Thickened gel compositions were prepared according to the mass ratio shown in Table 1, each composition including component (a): lecithin (soybean lecithin (trade name: "L-α-Phosphatidylcholine (Soy-95%)," available from Avanti Polar Lipids, Inc.)), component (b) : ascorbic acid (trade name: "L-(+)-ascorbic acid," available from Wako Pure Chemical Industries, Ltd.), and component (c) : n-decane (available from KANTO CHEMICAL CO., INC., 0.774 mPa·s (25°C)). The prepared compositions correspond to Examples 1 to 11.

(Preparation of thickened gel composition)

Component (a), lecithin, and component (b), ascorbic acid, were sealed in necessary amounts in a vial, and then methanol was added. The mixture was agitated with a magnetic stirrer. After lecithin and ascorbic acid were completely dissolved, methanol was completely evaporated under reduced pressure. A necessary amount of oil component (c) was added to the product, and the mixture was stirred overnight. The vial was settled for several days in a 25°C thermostat to stabilize the product. A thickened gel composition was prepared. Thickened gel compositions in Examples and Comparative Examples described below were prepared as above.

In Examples 1 to 11, thickened gel compositions were prepared with 5.0 to 20.0 mass% component (a), lecithin. In these thickened gel compositions, the proportion of ascorbic acid in the organogelling agent, i.e., component (b)/(component (a) + component (b)) was 7 to 16 mass%, and the proportion of the organogelling agent in the thickened gel composition, i.e., (component (a) + component (b))/(component (a) + component (b) + component (c)) was 5.7 to 24 mass%. The thickened gels were transparent or translucent, and were evaluated as rank AA (Gelation) or A (Thickening). The gels in Examples 2 and 6 evaluated as rank A (Thickening) were in practically usable levels without problems. The results of evaluation are shown in Table 1.

[Table 1]

| Thickened gel composition | | Examples | | | | | | | | | | |
|---|---|---|---|---|---|---|---|---|---|---|---|---|
| | | 1 | 2 | 3 | 4 | 5 | 6 | 7 | 8 | 9 | 10 | 11 |
| Formulation | Lecithin | 5 | 10 | 10 | 10 | 10 | 20 | 20 | 20 | 20 | 20 | 20 |
| | L-ascorbic acid | 0.7 | 1 | 1.3 | 1.5 | 1.75 | 1.5 | 2 | 2.5 | 3 | 3.5 | 4 |
| | 3-O-ethyl ascorbic acid | | | | | | | | | | | |
| | n-Decane | 94.3 | 89 | 88.7 | 88.5 | 88.25 | 78.5 | 78 | 77.5 | 77 | 76.5 | 76 |
| | Isopropyl myristate | | | | | | | | | | | |
| Evaluation | Thickening or gelation | AA | A | AA | AA | AA | A | AA | AA | AA | AA | AA |
| | Degree of transparency | AA | AA | AA | AA | A | AA | AA | AA | AA | AA | AA |

[Examples 12 to 21]

(Compounding of thickened gel composition)

Thickened gel compositions in Examples 12 to 21 were according to the mass ratio shown in Table 2, each composition containing component (a), lecithin (soybean lecithin (trade name: "L-α-Phosphatidylcholine (Soy-95%)," available from Avanti Polar Lipids, Inc.)), component (b), ascorbic acid (trade name: "L-(+)-ascorbic acid," available from Wako Pure Chemical Industries, Ltd.), and component (c), isopropyl myristate (available from Wako Pure Chemical Industries, Ltd., 4.74 mPa·s (25°C)).

In Examples 12 to 21, the proportion of ascorbic acid in the organogelling agent, i.e., component (b)/(component (a) + component (b)) was 16 to 24 mass%, and the proportion of the organogelling agent in the thickened gel composition, i.e., (component (a) + component (b))/(component (a) + component (b) + component (c)) was 6.2 to 26 mass%. The thickened gels were transparent or translucent, and were evaluated as rank AA (Gelation) or rank A (Thickening). The gels evaluated as rank A (Thickening) were in practically usable levels without problems. The results of evaluation are shown in Table 2.

[Table 2]

| Thickened gel composition | | Examples | | | | | | | | | |
|---|---|---|---|---|---|---|---|---|---|---|---|
| | | 12 | 13 | 14 | 15 | 16 | 17 | 18 | 19 | 20 | 21 |
| Formulation | Lecithin | 5 | 10 | 10 | 10 | 10 | 10 | 10 | 20 | 20 | 20 |
| | L-ascorbic acid | 1.2 | 2.25 | 2.4 | 2.5 | 2.6 | 2.7 | 2.75 | 4 | 5 | 6 |
| | 3-O-ethyl ascorbic acid | | | | | | | | | | |
| | n-Decane | | | | | | | | | | |
| | Isopropyl myristate | 93.8 | 87.75 | 87.6 | 87.5 | 87.4 | 87.3 | 87.25 | 76 | 75 | 74 |
| Evaluation | Thickening or gelation | A | A | A | AA | AA | AA | AA | A | AA | AA |
| | Degree of transparency | AA | AA | AA | AA | AA | AA | A | AA | AA | AA |

[Examples 22 to 24]

(Preparation of thickened gel composition)

Component (b), L-ascorbic acid, used in Examples 2 and 4 was replaced with 3-O-ethyl ascorbic acid (available from JUNSEI CHEMICAL CO., LTD.) to prepare thickened gel compositions in Examples 22 and 24. Component (b), L-ascorbic acid, used in Example 2 was replaced with 1.25 mass% 3-O-ethyl ascorbic acid to prepare a thickened gel composition in Example 23. Thickened gel compositions were prepared according to the formulation shown in Table 3.

The thickened gel compositions in Examples 22 to 24 were prepared with 10 mass% component (a), lecithin, and predetermined amounts of component (b), 3-O-ethyl ascorbic acid, and component (c), n-decane. The gels were evaluated as rank A (Thickening), and the degrees of transparency were evaluated as rank AA (Transparent). The results

of evaluation are shown in Table 3.

[Comparative Examples 1 and 2]

(Compounding of thickened gel composition)

A thickened gel composition in Comparative Example 1 was prepared according to a formulation containing 10 mass% component (a), lecithin (soybean lecithin (trade name: "L-α-Phosphatidylcholine (Soy-95%)," available from Avanti Polar Lipids, Inc.)), and 90 mass% component (c), n-decane (available from KANTO CHEMICAL CO., INC.). A thickened gel composition in Comparative Example 2 was prepared as in Comparative Example 1 except that component (c) was replaced with isopropyl myristate (available from Wako Pure Chemical Industries, Ltd.).

Comparative Examples 1 and 2 not containing component (b), ascorbic acid or an ascorbic acid derivative, did not form any thickened gel. The formulations and the results of evaluation are shown in Table 3. In contrast, in the present invention, the thickened gel had a viscosity 2500000 times that of n-decane as oil component (c) (0.774 mPa·s at 25°C), and had a viscosity 10000 times that of isopropyl myristate (4.74 mPa·s at 25°C) as shown in Examples. No thickened gel was produced from a combination of component (b), ascorbic acid or an ascorbic acid derivative and oil component (c).

[Table 3]

| Thickened gel composition | | Examples | | | Comparative Examples | |
|---|---|---|---|---|---|---|
| | | 22 | 23 | 24 | 1 | 2 |
| Formulation | Lecithin | 10 | 10 | 10 | 10 | 10 |
| | L-ascorbic acid | | | | | |
| | 3-O-ethyl ascorbic acid | 1 | 1.25 | 1.5 | | |
| | n-Decane | 89 | 88.75 | 88.5 | 90 | |
| | Isopropyl myristate | | | | | 90 |
| Evaluation | Thickening or gelation | A | A | A | F | F |
| | Degree of transparency | AA | AA | AA | F | F |

INDUSTRIAL APPLICABILITY

The present invention can provide an organogelling agent that can form reverse worm-like micelles with a ternary system of lecithin, ascorbic acid or an ascorbic acid derivative, and a variety of oils. The reverse worm-like micelles formed in the thickened gel composition have internal hydrophilic environments, and can encapsulate water-soluble components, drugs, and enzymes. The organogelling agent can be widely used as an organogelling agent for a variety of cosmetics, medicines, and foods. Furthermore, the thickened gel composition prepared from the organogelling agent has thixotropy, ease of handling, and high long-term stability.

## Claims

1. An organogelling agent for forming reverse worm-like micelles, comprising:

   lecithin (a); and
   ascorbic acid or an ascorbic acid derivative (b).

2. The organogelling agent according to claim 1, wherein the mixture contains ascorbic acid or an ascorbic acid derivative (b) in an amount of 0.1 to 35 mass% to the total mass of lecithin (a) and ascorbic acid or an ascorbic acid derivative (b).

3. A thickened gel composition, comprising:

   the organogelling agent according to claim 1 or 2; and

an oil component (c),
wherein the thickened gel composition contains reverse worm-like micelles.

**4.** The thickened gel composition according to claim 3, wherein the thickened gel composition is at least one composition selected from a cosmetic composition, a medicine composition, a food composition, a coating composition, an ink composition, and a lubricant composition.

**5.** The thickened gel composition containing oil component (c) according to claim 3 or 4, wherein the thickened gel composition contains 1 to 70 mass% organogelling agent.

**6.** A process for preparing an organogelling agent for forming reverse worm-like micelles, comprising:

dissolving lecithin (a) and ascorbic acid or an ascorbic acid derivative (b) in an organic solvent, and evaporating the organic solvent.

**7.** A process for preparing a thickened gel composition containing reverse worm-like micelles, comprising:

dissolving lecithin (a) and ascorbic acid or an ascorbic acid derivative (b) in an organic solvent, and evaporating the organic solvent to prepare an organogelling agent; and
mixing an oil component (c) with the organogelling agent.

FIG.1

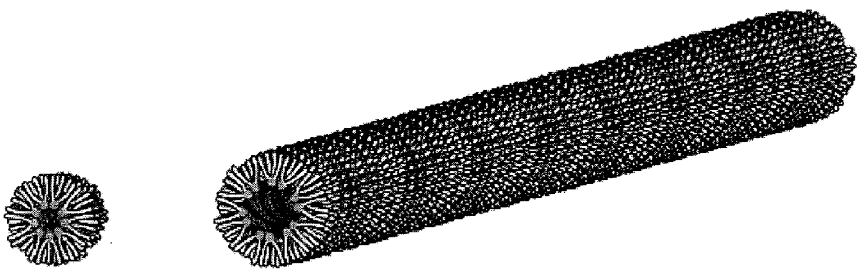

The reverse spherical micelle    The reverse worm-like micelle

**INTERNATIONAL SEARCH REPORT**

International application No. PCT/JP2012/081125

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C09K3/00, A23L1/03, A61K8/02, A61K8/55, A61K8/67, A61K9/06, A61K47/14, A61K47/22, A61K47/24, C09D5/04, C09D7/12, C09D201/00, C10M141/10, C10M129/20, C10M137/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
JSTPlus/JMEDPlus/JST7580(JDreamII), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2009-137874 A (Kabushiki Kaisha Authele), 25 June 2009 (25.06.2009), claims; paragraphs [0030] to [0041] (Family: none) | 1-5<br>6-7 |
| X<br>A | JP 2008-063280 A (Cedarcos Japan Co., Ltd.), 21 March 2008 (21.03.2008), claims; paragraphs [0014] to [0023] (Family: none) | 1-2<br>3-7 |
| X<br>A | JP 2001-226293 A (Kotaro Pharmaceutical Co., Ltd.), 21 August 2001 (21.08.2001), claims; paragraphs [0011], [0013], [0030] to [0060] (Family: none) | 1-2<br>3-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>30 January, 2013 (30.01.13) | Date of mailing of the international search report<br>12 February, 2013 (12.02.13) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2012/081125

C (Continuation). DOCUMENTS CONSIDERED TO BE RELEVANT

| Category* | Citation of document, with indication, where appropriate, of the relevant passages | Relevant to claim No. |
|---|---|---|
| X<br>A | Oxidation in fish-oil-enriched mayonnaise, European Food Research and Technology, 2000, 210, 242-257 | 1-5<br>6-7 |

Form PCT/ISA/210 (continuation of second sheet) (July 2009)

**INTERNATIONAL SEARCH REPORT**

International application No.
PCT/JP2012/081125

Continuation of A. CLASSIFICATION OF SUBJECT MATTER
(International Patent Classification (IPC))

*C09K3/00*(2006.01)i, *A23L1/03*(2006.01)i, *A61K8/02*(2006.01)i, *A61K8/55*(2006.01)i, *A61K8/67*(2006.01)i, *A61K9/06*(2006.01)i, *A61K47/14*(2006.01)i, *A61K47/22*(2006.01)i, *A61K47/24*(2006.01)i, *C09D5/04*(2006.01)i, *C09D7/12*(2006.01)i, *C09D201/00*(2006.01)i, *C10M141/10*(2006.01)i, *C10M129/20*(2006.01)n, *C10M137/04*(2006.01)n

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5004911 A
- WO 2010082487 A
- WO 2010122694 A
- JP 2010270299 A


### Non-patent literature cited in the description


- **P. L. LUISI et al.** *Colloid & Polymer Science,* 1990, vol. 268, 356
- **YU. A. SHCHIPUNOV.** *Colloids and Surfaces A,* 2001, vol. 183-185, 541
- **S. H. TUNG et al.** *Journal of the American Chemical Society,* 2006, vol. 128, 5751